# FASCICULE DE BREVET EUROPEEN

(11) **EP 1 058 558 B1**
(45) Date de publication et mention de la délivrance du brevet: **26.01.2005**
(21) Numéro de dépôt: 99937879.7
(22) Date de dépôt: 01.03.1999
(51) Int. Cl.: A61K 39/39, A61K 48/00

(54) **Vaccins recombinants et adjuvés contre le virus de l'influenza et le virus de l'herpès**
Adjuvierte rekombinante Lebendimpfstoffe gegen Influenza- oder Herpesviren
Adjuvated, live recombinant vaccines against influenza- and herpesviruse

(30) Priorité: 03.03.1998 FR 9802800
(43) Date de publication de la demande: 13.12.2000
(73) Titulaire: MERIAL, 69002 Lyon (FR)
(72) Inventeur: AUDONNET, Jean-Christophe, Francis, F-69006 Lyon (FR); MINKE, Jules, Maarten, F-69960 Corbas (FR)
(74) Mandataire: Nargolwalla, Cyra
(86) Numéro de dépôt international: PCT/FR1999/000453
(87) Numéro de publication internationale: WO 1999/044633

(56) Documents cités:
- EP-A- 0 283 085
- EP-A- 0 532 833
- EP-A- 0 786 518
- WO-A-94/16681
- WO-A-97/49825
- WO-A-98/03198
- US-B- 4 920 213
- US-B- 5 820 869
- Haanes et al: 'The bovine parainfluenza virus type-3 (BPIV-3) hemagglutinin/neuraminidase glycoprotein expressed in baculovirus protects calves against experimental BPIV-3 challenge', 1997, Vaccine, vol. 15, No.6/7, pp. 730-738.*r*n
- Pialoux et al: 'A prime boost to HIV preventive vaccine using a recombinant canarypox virus expressing glycoprotein 160 (MN) followed by a recombinant glycoprotein 160 (MN/LAI)', 1995, Aids Research and Human Retroviruses, vol. 11, no. 3, pp. 373-381.
- Chambers et al: 'Protection of Chickens from Lethal Influenza Infection by Vaccinia-Expressed Hemagglutinin', Virology, 167, 414-421 (1988)

## Description

La présente invention concerne un perfectionnement aux vaccins vivants recombinants intégrant et exprimant in vivo un ou plusieurs gènes hétérologues. Elle a trait en particulier à de tels vaccins adjuvés, à l'utilisation de composés adjuvants particuliers pour la mise en oeuvre de tels vaccins ainsi qu'aux méthodes de vaccination y relatives. Elle a encore pour objet un procédé de préparation de ces vaccins.

Il est classique d'incorporer aux vaccins inactivés ou de sous-unités des adjuvants destinés à accroître la réponse immunitaire vis-à-vis des antigènes que comprennent ces vaccins.

On a aussi pensé incorporer des adjuvants aux vaccins vivants atténués lorsque l'atténuation de microorganismes a conduit à une diminution de la réponse immunitaire.

Récemment il a aussi été proposé des vaccinations combinées contre plusieurs pathogènes à l'aide d'un vaccin inactivé pour une valence et d'un vaccin vivant atténué pour l'autre valence. On a ainsi proposé de reprendre le vaccin vivant atténué, conservé sous forme lyophilisée, dans la composition de vaccin inactivé adjuvé. Celle-ci joue le rôle de véhicule de reprise pour le vaccin vivant, sans qu'aucun effet adjuvant ne soit recherché pour ce dernier.

EP-A-532 833 propose un vaccin contre la rhinopneumonie du cheval, pathologie provoquée par l'herpès virus équin (EHV). Le vaccin est un vaccin inactivé et adjuvé, regroupant virus EHV-1 et virus EHV-4 inactivés, adjuvés par l'adjuvant Havlogen®, à base de polymère polyacrylique.

Comme pour la plupart des herpèsvirus, il n'existe actuellement aucun vaccin efficace permettant l'élimination rapide du virus après l'infection. Les vaccins connus tentent de protéger contre l'apparition des signes cliniques. En général, l'effet sur l'excrétion virale reste limité.

Selon EP-A-532 833, le vaccin développé conduirait à une chute de l'excrétion virale allant de 79 à 93 % (voir partie résultats). Huit animaux témoins sur neuf ont excrété du virus après épreuve sur une moyenne de 1,4 jours alors que la durée d'excrétion normale après épreuve est habituellement supérieure ou égale à 5 jours. Cela traduit une épreuve de faible intensité qui majore artificiellement la protection des chevaux vaccinés par rapport aux témoins. La baisse de 1 excrétion virale n'est donc pas significative dans cette expérience.

Des adjuvants de type carbomère (carbomer) ont aussi été utilisés dans des vaccins influenza équins (IEV) à virus inactivé.

Mumford et al. (Epidemiol. Infect. (1994), 112, 421-437) rappellent qu'il faut deux doses de vaccin IEV équin pour induire chez le cheval une réponse humorale transitoire et une faible protection contre le virus. Les auteurs comparent les effets adjuvants du carbomère et du phosphate d'aluminium sur des vaccins inactivés en présence ou non d'anatoxine tétanique. Dans tous les cas, un faible titre en anticorps mesuré par la technique SRH (single radial haemolysis) vis-à-vis des souches H7N7 et H3N8 est obtenu après une première vaccination et il faut attendre une deuxième, puis une troisième vaccination pour voir apparaître des réponses transitoires plus fortes.

US-A-4 500 513 présente aussi des essais de vaccination contre le virus influenza équin avec un vaccin inactivé en présence d'un carbomère. L'origine des animaux et leur statut médical n'est pas indiqué avec précision et il semble qu'il s'agisse d'animaux du terrain (colonne 11, 2ème paragraphe). Les titres élevés en anticorps, mesurés par une technique d'inhibition de l'hémagglutination, indiquent que les animaux avaient probablement déjà été infectés par influenza et que la réponse induite après vaccination était de type rappel, et non pas de primo-vaccination

Enfin, Fort Dodge Solvay commercialise des vaccins influenza équin inactivé (Dwaxyn® IE et IE-T plus) et un vaccin rhinopneumonie équine inactivé (Duvaxyn® EHV_{1,4}), en adjuvant carbomère.

On connaît aussi des vaccins inactivés commerciaux contre l'influenza équin, adjuvés par l'hydroxyde d'alumine (par exemple Tetagripiffa®, Mérial, Lyon, France).

Un grand nombre d'autres adjuvants sont utilisés dans le cadre des vaccins classiques inactivés ou de sous-unités (voir par exemple E. J. Haanes et al., Vaccine 1997, 15, 617:730-738). On peut citer par exemple hydroxyde d'alumine, phosphate d'alumine, Avridine®, DDA, manophosphoryl lipid A, Pluronic L121 et autres polymères-blocs, muramyl peptides, saponines, trehalose dimycolate, copolymères d'anhydride maléique et d'éthylène, copolymères de styrène et d'acide acrylique ou méthacrylique, polyphosphazène, émulsions huileuses, etc.

G. Pialoux et al., AIDS Research and Human Retroviruses 1995, 11,3 : 373-381, décrivent un protocole prime-boost comprenant une primo-administration avec un vaccin à base d'un vecteur canarypox exprimant gp160 de HIV, puis un rappel avec un vaccin de sous-unité à base de gp160. Seul ce dernier est adjuvé.

WO-A-97/49825 décrit un vaccin vivant recombinant, utilisant l'herpèsvirvs canin (CHV) comme vecteur pour l'expression *in vivo* d'un antigène d'un agent pathogène canin, e.g. antigène du virus de la maladie de Carré, du virus de la rage ou du virus parainfluenza de type 2. Ce document n'envisage pas l'ajout d'un adjuvant. Il existe d'ailleurs peu d'exemples concrets de vaccin de ce type formulés avec un adjuvant.

WO-A-94 16689 suggère d'adjuver un vaccin vivant recombinant exprimant un gène hétérologue d'un virus enveloppé par une composition vaccinale sous forme d'émulsion eau-dans-l'huile, huile-dans-l'eau ou eau-dans-l'huile-dans l'eau.

Une telle solution peut cependant présenter un certain nombre d'inconvénients.

En pratique, l'utilisateur final doit disposer d'une part de principe actif lyophilisé et d'autre part d'une émulsion déjà constituée, qui doit permettre de reprendre le principe actif lyophilisé.

Un manque de stabilité de l'émulsion au cours du stockage pourrait être préjudiciable à l'efficacité et à l'innocuité du vaccin reconstitué.

L'activité des microorganismes vivants atténués peut être remise en cause à la suite de leur instabilité dans la phase huileuse. Cela peut notamment être le cas pour les virus qui peuvent alors perdre leur viabilité.

Les vaccins en émulsion peuvent aussi poser des problèmes d'innocuité au site d'injection.

La présente invention s'est donc donnée pour objectif de proposer de nouvelles compositions vaccinales à base de vaccin vivant recombinant exprimant au moins une séquence nucléotidique hétérologue, notamment gène hétérologue, comportant un adjuvant qui soit capable d'accroître de façon remarquable l'immunité conférée par rapport au même vaccin non adjuvé et qui soit parfaitement adapté à ce type de vaccin.

La déposante a trouvé que les composés de la classe des carbomères étaient en mesure d'adjuver dans les conditions requises ce type de vaccin et cela dans des proportions inattendues. Des essais menés sur les herpèsvirus animaux (EHV-1, Equine Herpes Virus) ont montré que l'apport du carbomère permettait de diminuer l'excrétion virale lors d'une infection expérimentale, dans des proportions inattendues. D'autres essais menés sur le virus Influenza A équin ont permis d'obtenir de manière surprenante, chez le cheval, des titres sérologiques précoces et très élevés, supérieurs à ceux obtenus avec les meilleurs vaccins commerciaux.

La présente invention a donc pour objet un vaccin vivant recombinant comprenant un vecteur viral incorporant et exprimant in vivo une séquence nucléotidique hétérologue sélectionnée parmi un gène d'un herpès virus animal et un gène d'un virus influenza, et au moins un composé adjuvant choisi parmi les polymères de l'acide acrylique ou méthacrylique et les copolymères d'anhydride maléique et de dérivé alcényle.

Les composés préférés sont les polymères de l'acide acrylique ou méthacrylique réticulés, notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 (incorporé par référence) décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol Parmi eux, on peut citer les Carbapol® 974P, 934P et 971 P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 186 : 778-780, 4 juin 1960, incorporé par référence.

Sur le plan de leur structure, les polymères d'acide acrylique ou méthacrylique et les EMA® sont formés de préférence de motifs de base de formule suivante : 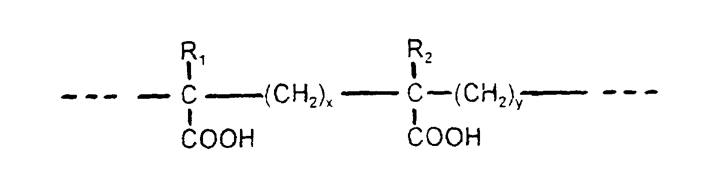 dans laquelle :
- R₁ et R₂, identiques ou différents, représentent H ou CH₃
- x = 0 ou 1, de préférence x = 1
- y = 1 ou 2, avec x + y = 2

Pour les EMA®, x = 0 et y = 2. Pour les carbomères, x = y = 1.

La dissolution de ces polymères dans l'eau conduit à une solution acide qui sera neutralisée. de préférence jusqu'à pH physiologique, pour donner la solution adjuvante dans laquelle le vaccin proprement dit sera incorporé. Les groupes carboxyliques du polymère sont alors en partie sous forme COO⁻.

De manière préférée, on réalise une solution d'adjuvant selon l'invention, notamment de carbomère, dans de l'eau distillée, de préférence en présence de chlorure de sodium, la solution obtenue étant à pH acide. On dilue cette solution mère en l'ajoutant dans la quantité nécessaire (pour l'obtention de la concentration finale souhaitée), ou une partie importante de celle-ci, d'eau chargée en NaCl, de préférence eau physiologique (NaCl 9 g/l), en une ou plusieurs fois avec neutralisation concomitante ou subséquente (pH 7,3 à 7,4), de préférence par NaOH. Cette solution à pH physiologique sera utilisée telle quelle pour reprendre le vaccin, notamment conservé sous forme lyophilisée.

La concentration en polymère dans la composition vaccinale finale sera de 0,01 % à 2 % PN, plus particulièrement de 0,06 à 1 % P/V, de préférence de 0,1 à 0,6 % PN.

L'invention se révèle particulièrement utile pour la vaccination contre les herpèsvirus animaux. L'invention vise tout particulièrement les herpèsvirus équin (EHV-1 et EHV-4 notamment), félin (FHV), canin (CHV), aviaire (Marek et ILTV), bovin (BHV), porcin (PRV = virus de la maladie d'Aujeszky ou virus de la pseudo-rage).

L'invention a donc pour objet des vaccins vivants recombinants comprenant au moins un vecteur virai incorporant et exprimant au moins un gène d'un tel herpèsvirus, et au moins un adjuvant conforme à l'invention.

A titre d'exemple, l'homme de l'art peut se reporter à WO-A-92 15672 (incorporé par référence), qui décrit la réalisation de vecteurs d'expression à base de poxvirus capables d'exprimer de tels gènes. On y trouvera par exemple un canarypox exprimant les gènes gB, gC et gD de EHV-1 (vCP132), ce qui est applicable aussi à EHV-4, un virus de la vaccine exprimant ces mêmes gènes (vP 1043), un virus de la vaccine exprimant les gènes gl(gB), glll(gC), gIV(gD) de 8HV-1, un canarypox exprimant gD de FHV-1, ou encore des recombinants exprimant les gènes gII (gB), glll (gC), gp50 (gD) de PRV. Il peut aussi se reporter à WO-A-95/26 751 (incorporé par référence), qui décrit des virus recombinants vCP320, vCP322 et vCP294 exprimant les gènes gB, gC, gD, respectivement. de CHV. Il peut aussi se reporter aux recombinants exprimant des gènes de FHV, PRV, BHV dans FR-A-2 647 808 ou WO90/12882 (incorporés par référence).

L'invention se révèle aussi particulièrement intéressante pour la vaccination contre les virus Influenza, comme on le démontre ici pour EIV (virus influenza équin ou virus de la grippe équine). On peut aussi citer la grippe aviaire (AIV), la grippe porcine (Swine influenza virus).

A titre d'exemple, l'homme de l'art peut se reporter au canarypox recombinant exprimant le gène HA d'EIV dans WO-A-92 15 672.

L'invention a donc pour objet des vaccins vivants recombinants comprenant au moins un vecteur viral incorporant et exprimant au moins un gène d'un tel virus influenza, et au moins un adjuvant conforme à l'invention. Notamment, le vaccin comprend un mélange de deux ou trois vecteurs incorporant et exprimant chacun un gène HA, les gènes provenant de souches différentes, oar exemple des souches influenza équin (grippe équine) Prague, Kentucky et Newmarket. Dans le même ordre d'idée, un seul vecteur peut être utilisé pour incorporer et exprimer les HA de 2 ou des 3 souches.

L'invention s'applique aussi à d'autres pathogènes animaux, tels que notamment FeLV (voir aussi recombinants canarypox dans WO-A-92 15672 à titre d'exemple, exprimant env, gag = vCP93 et vCP97), le tétanos (voir aussi WO-A-92 15 672 et les recombinants vCP161 et vP1075, canarypox et vaccine, exprimant la toxine tétanique), virus de la maladie de Carré (canine distemper virus ou CDV) (voir recombinant vCP 258 dans WO-A-95 27780, incorporé par référence).

L'invention a donc pour objet des vaccins vivants recombinants comprenant au moins un vecteur viral incorporant et exprimant au moins un gène d'un tel virus.

L'invention a aussi pour objet des vaccins recombinants multi-valents, c'est-à-dire contenant deux ou plusieurs vecteurs recombinants exprimant des antigènes de deux ou plusieurs maladies, en mélange dans une solution adjuvante conforme à l'invention.

Au demeurant, l'invention s'applique à l'utilisation de tout type de vecteur vira! d'expression, tel que poxvirus (virus de la vaccine, y compris NYVAC selon WO-A-92/15672, fowlpox. canarypox, pigeonpox, swinepox. etc.), adénovirus, herpèsvirus. Le canarypox, e.g. ALVAC (WO-A-95/27780 et WO-A-92/15672), se révèle particulièrement approprié dans le cadre de la présente invention.

Dans le vaccin prêt-à-l'emploi, notamment reconstitué, le vecteur viral est présent dans les quantités habituellement utilisées et décrites dans la littérature.

Les vaccins vivants recombinants se présentent en général sous une forme lyophilisée permettant leur conservation et sont repris extemporanément dans un solvant ou excipient, qui sera ici la solution d'adjuvant conforme à l'invention.

L'invention a donc aussi pour objet un ensemble de vaccination comprenant, conditionnés séparément, du vaccin lyophilisé et une solution du composé adjuvant selon l'invention pour la reprise du vaccin lyophilisé.

L'invention a aussi pour objet une méthode de vaccination consistant à administrer par voie parentérale, de préférence sous-cutanée, intramusculaire, intradermique, ou par voie mucosale, un vaccin conforme à l'invention, à raison d'une ou plusieurs administrations, éventuellement avec une étape préalable de reprise du vaccin lyophilisé (le vecteur recombinant) dans une solution de composé adjuvant.

Un objectif d'une telle méthode peut être de protéger les animaux sur le plan clinique et de diminuer l'excrétion virale, ce qui correspond surtout au cas des herpèsvirus.

Un autre objectif peut être d'accroître la réponse immunitaire et de la rendre plus précoce, notamment par induction d'anticorps dès la première administration.

L'invention a encore pour objet l'utilisation des composés adjuvants conformes à l'invention pour la réalisation de vaccins vivants recombinants, notamment conférant une réponse immunitaire améliorée et plus précoce et'ou une baisse accrue de l'excrétion virale. On se référera à ce qui a été dit plus haut.

L'invention va être maintenant décrite plus en détails, à l'aide de modes de réalisation pris à titre d'exemples non limitatifs et se référant au dessin dans lequel:
- la figure 1 représente la séquence nucléotidique du gène EIV HA de la souche EIV Newmarket 2/93 ;
- la figure 2 représente la séquence nucléotidique du gène FHV-1 gC ;

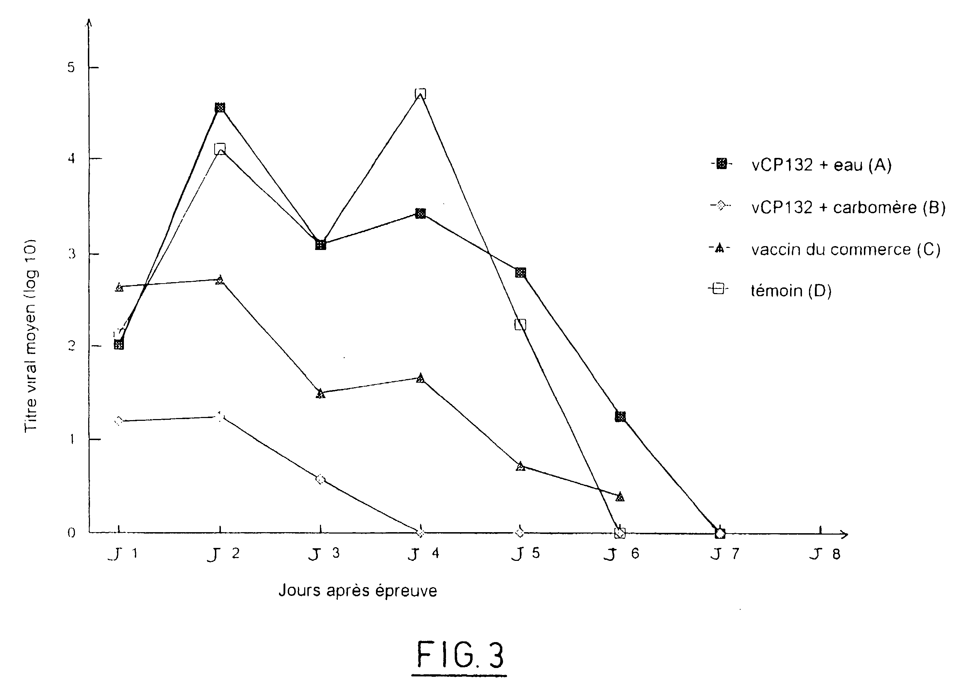

- la figure 3 représente un graphe montrant l'évolution de l'excrétion virale après infection expérimentale chez des chevaux vaccinés à l'aide de différents vaccins contre l'herpèsvirus équin

### EXEMPLES

### Exemple 1 : Génération des plasmides donneurs pour les sites d'insertion C3, C5 et C6 dans le virus canarypox "ALVAC"

Les plasmides donneurs pour les différents sites d'insertion dans le virus canarypox "ALVAC"(Tartaglia *et al.* Virology. 1992. **188**. 217-232 incorporé par référence), sont décrits dans la demande WO-A-95/27780, exemple 20.

Ces plasmides ont été désignés dans cette demande de la façon suivante :
"plasmide VQH6CP3LSA.2" pour le site "C3"
"plasmide HC5LSP28" pour le site "C5"
"plasmide pC6L" pour le site "C6".

### Exemple 2 : Génération du virus recombiné vCP258 (ALVAC/CDV HA+F)

La souche Onderstepoort du virus CDV a été utilisée pour isoler les gènes HA et F. (Séquence du gène HA décrite par Curran *et al.* Virology. 1991. **72**. 443-447, et séquence du gène F décrite par Barrett *et al.* Virus Research. 1987. **8.** 373-386, incorporés par référence).

La construction du plasmide donneur pMM103 pour l'insertion des cassettes d'expression promoteur vaccine H6- gène CDV F et promoteur vaccine H6-gène CDV HA dans le locus C6 du virus ALVAC est décrite dans l'exemple 19 de la demande WO-A-95/27780.

Ce plasmide a été utilisé comme plasmide donneur pour la recombinaison in *vitro* (Piccini et al. Methods in Enzymology. 1987. **153**. 545-563, incorporé par référence) avec le virus ALVAC pour générer le virus recombiné désigné vCP258 comme à l'exemple 19 de la susdite demande.

### Exemple 3 : Génération du virus recombiné vCP1502 (ALVAC/EIV HA Prague)

La séquence du gène HA (souche EIV Prague) est présentée à la figure N° 23 de la demande WO-A-92/15672. L'ARN viral du génome du virus de la grippe équine souche Prague 56 a été extrait à partir de 100 µl d'une suspension virale de ce virus avec le kit d'extraction "Total RNA Separator kit" de CLONTECH (Palo Alto, CA)(Cat#K1042-1). Le culot d'ARN a été repris dans 10 µl d'eau ultrapure et une réaction de synthèse d'ADN complémentaire, suivie d'une amplification PCR (= réaction "RT-PCR") a été réalisée en prenant comme matrice 2 µl d'ARN viral purifié et les oligonucléotides suivants : 
TAY51A (SEQ ID N°1) (70 mer)
5'CGCGGCCATCGCGATATCCGTTAAGTTTGTATCGTAATGAACACTCAAATTCT
AATATTAGCCACTTCGG 3'
et TAY53A (SEQ ID N°2) (36 mer)
5' CGCGCGGCGGTACCTTATATACAAATAGTGCACCGC 3'
 pour amplifier le gène EIV HA Prague. Le fragment PCR ainsi obtenu a été ligaturé dans le vecteur pCRII (InVitrogen, San Diego, CA) pour donner le plasmide pJT007.

Le plasmide pJT007 a été digéré par NruI et Asp718 pour isoler un fragment Nrul-718 d'environ 1800 pb contenant la fin du promoteur H6 et le gène HA Prague 56 dans sa totalité. Ce fragment a été ligaturé avec le plasmide donneur C5 HC5LSP28, préalablement digéré par Nrul et Asp718, pour donner finalement le plasmide pJT008. Ce plasmide contient la cassette d'expression H6-gène HA Prague 56 dans le locus C5 du virus ALVAC. La structure de ce plasmide a été vérifiée par séquençage et carte de restriction complète.

Ce plasmide est le plasmide donneur pour l'insertion de la cassette d'expression H6-gène HA Prague 56 dans le locus C5.

Après linéarisation par Notl, le plasmide pJT008 a été utilisé comme plasmide donneur pour la recombinaison *in vitro* (Piccini *et al.* Methods in Enzymology. 1987. 153. 545-563) avec le virus ALVAC pour générer le virus recombiné désigné **vCP1502.**

### Exemple 4 : Génération du virus recombiné vCP1529 (ALVAC/EIV HA Kentucky 1/94)

L'ARN viral du génome du virus de la grippe équine souche Kentucky 1/94 (Daly *et al.* J. Gen. Virol. 1996, **77**, 661-671, incorporé par référence) a été extrait à partir de 100 µl d'une suspension virale de ce virus avec le kit d'extraction "Total RNA Separator kit" de CLONTECH (Palo Alto, CA)(Cat#K1042-1). Le culot d'ARN a été repris dans 10 µl d'eau ultrapure et une réaction RT-PCR a été réalisée en prenant comme matrice 2 µl d'ARN viral purifié et les oligonucléotides suivants : 
TAY55A (SEQ ID N°3) (70 mer)
5'CGCGGCCATCGCGATATCCGTTAAGTTTGTATCGTAATGAAGACAACCATTAT
TTTGATACTACTGACCC 3'
et TAY57A (SEQ ID N°4) (42 mer)
5' CGCGCGGCGGTACCTCAAATGCAAATGTTGCATCTGATGTTG 3'
 pour amplifier le gène HA. Le fragment PCR ainsi obtenu a été ligaturé dans le vecteur pCRII (InVitrogen, San Diego, CA) pour donner le plasmide pJT001. La séquence du gène EIV HA souche Kentucky 1/94 cloné dans le plasmide pJT001 n'est pas différente de la séquence du gène EIV HA souche Kentucky 1/94 cisponible dans la base de données GenBank (Numéro d'accession L39914, incorporé par référence).

Le plasmide pJT001 contenant le gène HA (Kentucky 1/94) a été digéré par Nrul et Asp718 pour isoler un fragment Nrul-Asp718 de 1800 pb (contenant la fin du promoteur H6 et le gène HA Kentucky 1/94 dans sa totalité). Ce fragment a été ligaturé avec le plasmide donneur C5 HC5LSP28, préalablement digéré par Nrul et Asp718, pour donner finalement le plasmide pJT005. Ce plasmide contient la cassette d'expression H6-gène HA Kentucky 1/94 dans le locus C5 du virus ALVAC. La structure de ce plasmide a été vérifiée par séquençage et carte de restriction complète.

Ce plasmide est le plasmide donneur pour l'insertion de la cassette d'expression H6-gène HA Kentucky 1/94 dans le locus C5.

Après linéarisation par Notl, le plasmide pJT005 a été utilisé comme plasmide donneur pour la recombinaison *in vitro* (Piccini *et al.* Methods in Enzymology. 1987. **153.** 545-563) avec le virus ALVAC pour générer le virus recombiné désigné vCP1529.

### Exempte 5 : Génération du virus recombiné vCP1533 (ALVAC/EIV HA Newmarket 2/93)

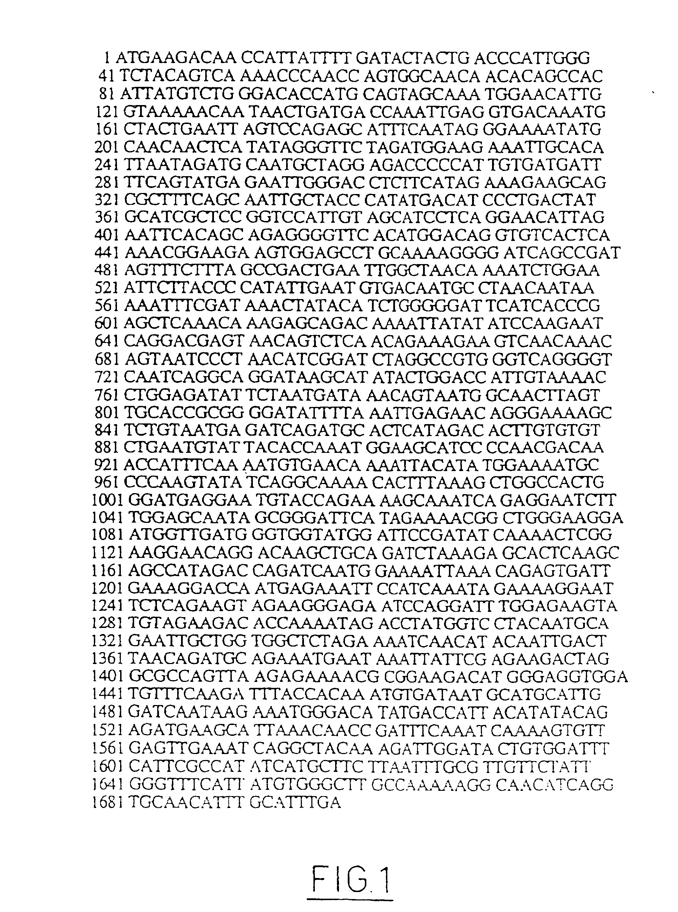

L'ARN viral du génome du virus de la grippe équine souche Newmarket 2/93 (Daly *et al.* J. Gen. Virol. 1996. **77.** 661-671). a été extrait à partir de 100 µl d'une suspension virale de ce virus avec le kit d'extraction "Total RNA Separator kit" de CLONTECH (Cat#K1042-)). Le culot d'ARN a été repris dans 10 µl d'eau ultrapure et une réaction RT-PCR a été réalisée en prenant comme matrice 2 µl d'ARN viral purifié et les oligonucléotides suivants : 
CCL007 (SEQ ID N°5) (40 mer)
5' TTGTCGACTCAATCATGAAGACAACCATTATTTTGATACT 3'
et CCL0018 (SEQ ID N°6) (34 mer)
5' TTGGATCCTTACTCAAATGCAAATGTTGCAYCTG 3'
 pour amplifier le gène HA. Le fragment PCR ainsi obtenu a été ligaturé dans le vecteur pCRII (InVitrogen, San Diego, CA) pour donner le plasmide pCCL026. La séquence du gène HA (souche EIV Newmarket 2/93) est présentée dans la figure N°1 (SEQ ID N°7).

Le plasmide pCCL026 contenant le gène HA (souche Newmarket 2/93) a été digéré par Spel et Accl. Les oligonucléotides suivants : 
TAY99N (SEQ ID N°8) (74 mer)
5'CTAGTTCGCGATATCCGTTAAGTTTGTATCGTAATGAAGACAACCATTATTTTG
ATACTACTGACCCATTGGGT 3'
et TAY100N (SEQ ID N°9) (72 mer)
5'AGACCCAATGGGTCAGTAGTATCAAAATAATGGTTGTCTTCATTACGATACAAA
CTTAACGGATATCGCGAA 3'
 ont été appariés et ligaturés avec le plasmide pCCL026 digéré par Spel+Accl pour donner le plasmide pJT003. L'oligonucléotide double brin TAY99N/TAY100N contient la région 3' du promoteur H6 jusqu'au site Nrul et les 40 premières bases codantes du gène HA.

Le plasmide JT003 a été digéré par Nrul et Xhol pour isoler un fragment Nrul-Xhol d'environ 1800 pb contenant la fin du promoteur H6 et le gène HA dans sa totalité. Ce fragment a été ligaturé avec le plasmide donneur C5 HC5LSP28, préalablement digéré par Nul et Xhol, pour donner finalement le plasmide pJT004. Ce plasmide contient la cassette d'expression H6-gène HA Newmarke:: 2/93 dans le locus C5 du virus ALVAC. La structure de ce plasmide a été vérifiée par séquençage et carte de restriction complète.

Ce plasmide est le plasmide donneur pour l'insertion de la cassette d'expression H6-gène HA Newmarket 2/93 dans le locus C5.

Après linéarisation par Pvul, le plasmide pJT004 a été utilisé comme plasmide donneur pour la recombinaison *in vitro* (Piccini *et al.* Methods in Enzymology. 1987. **153**. 545-563) avec le virus ALVAC pour générer le virus recombiné désigné **vCP1533**.

### Exemple 6 : Génération du virus recombiné vCP132 (ALVAC/EHV-1 gB+gC+gD)

La construction du virus recombiné est décrite dans les exemples 25 et 26 de la demande WO-A-92/15672. Ce virus a été généré par recombinaison *in vitro* entre le virus ALVAC et le plasmide donneur pJCA049. Ce plasmide contient les 3 cassettes d'expression suivantes clonées dans le site d'insertion C3 :
promoteur vaccine I3L-gène EHV-1 gB
promoteur vaccine H6-gène EHV-1 gC
promoteur entomopox 42K-gène EHV-1 gD

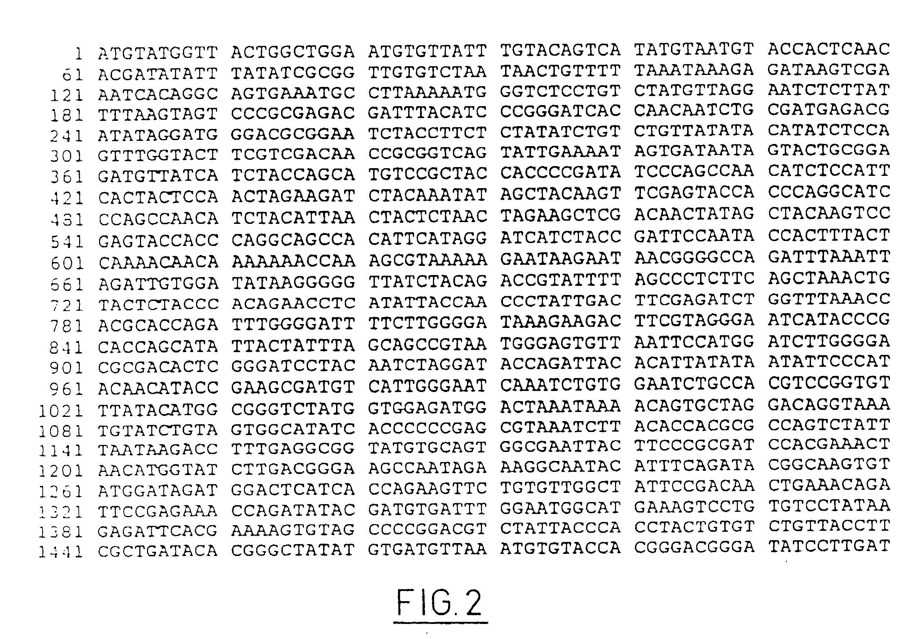

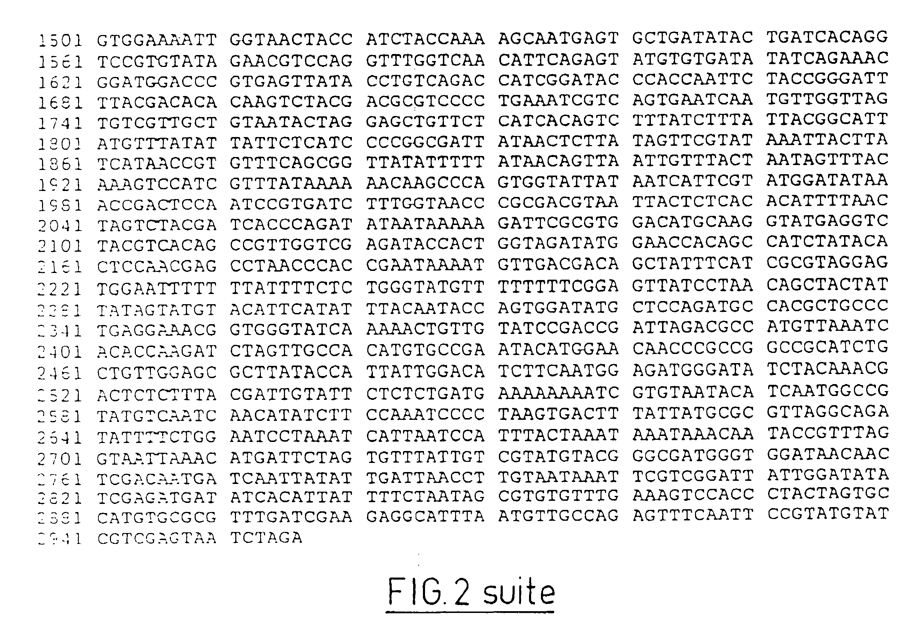

Les séquences des gènes EHV-1 gB, gC et gD sont décrites dans la demande WO-A-92/15672 aux figures N° 2 (séquence du gène EHV-1 gp13 = gC), N°6 (séquence du gène EHV-1 gp14 = gB) et N°12 (séquence des gènes EHV-1 gD, gp63 et gE).

### Exemple 7 : Génération du virus recombiné vCP243 (ALVAC/FHV-1 gB+gC+gD)

La séquence du gène FHV-1 gB (souche CO) est présentée à la figure N°34 de la demande WO-A-90/12882.

Le gène FHV-1 gC (souche CO)(séquence présentée à la figure N°2) (SEQ ID N°10) a été cloné à partir du fragment EcoRI F (7,6 kpb). Il a une taille de 1599 pb et code pour une protéine de 533 acides aminés.

Le gène FHV-1 gD (souche CO)(séquence présentée à la figure N°28 de la demande WO-A-92/15672) a été cloné à partir du fragment EcoRI M (4,4 kpb) (plasmide pFHVEcoRIM).

**Construction de la cassette d'expression I3L-gène FHV-1 gB muté au niveau des signaux d'arrêt précoce de transcription (TTTTTNT).**

Les oligonucléotides suivants : 
MP287 (SEQ ID N°11)  (20 mer)
5' GATTAAACCTAAATAATTGT 3'
et JCA158 (SEQ ID N°12)  (21 mer)
5' TTTTTCTAGACTGCAGCCCGGGACATCATGCAGTGGTTAAAC 3'
 ont été utilisés pour une amplification PCR avec la matrice d'un plasmide contenant le promoteur vaccine I3L (Rivière *et al.* J. Virol. 1992. **66**. 3424-3434, incorporé par référence) pour générer un fragment Xbal-bout franc de 120 pb (contenant le promoteur vaccine 13L) = fragment A. Les oligonucléotides suivants : 
JCA213 (SEQ ID N°13) (18 mer)
5' GGGTTTCAGAGGCAGTTC 3'
et JCA238 (SEQ ID N°14) (21 mer)
5' ATGTCCACTCGTGGCGATCTT 3'
 ont été utilisés pour générer par PCR à partir de la matrice du plasmide pJCA001 un fragment bout franc-BamHI de 720 pb (contenant la partie 5' du gène FHV-1 gB) = fragment B.

Le fragment A a été digéré par Xbal, puis phosphorylé. Le fragment B a été digéré par BamHI, puis phosphorylé. Les fragments A et B ont ensuite été ligaturés ensemble avec le vecteur pBluescript SK+, préalablement digéré par Xbal et BamHl, pour donner le plasmide pJCA075.

Les oligonucléotides suivants : 
JCA158 (SEQ ID N°15) et JCA211 (SEQ ID N°16) (21 mer):
5' GTGGACACATATAGAAAGTCG 3'
 ont été utilisés pour générer par PCR à partir de la matrice du plasmide pJCA075 un fragment Xbal-bout franc de 510 pb (contenant le promoteur 13L fusionné à la partie 5' du gène FHV-1 gB mutée au niveau du signal TTTTTNT) = fragment C.

Les oligonucléotides suivants : 
JCA212 (SEQ ID N°17) (21 mer)
5' CACCTTCAGGATCTACTGTCG 3'
et JCA213 (SEQ ID N°13) (18 mer)
 ont été utilisés pour générer par PCR à partir de la matrice du plasmide pJCA001 un fragment bout franc-BamHl de 330 pb (contenant la partie centrale du gène FHV-1 gB) = fragment D.

Le fragment C a été digéré par Xbal, puis phosphorylé. Le fragment D a été digéré par BamHl, puis phosphorylé. Les fragments C et D ont alors été ligaturés ensemble avec le vecteur pBluescript SK+, préalablement digéré par Xbal et BamHl, pour donner le plasmide pJCA076.

Les oligonucléotides suivants : 
JCA239 (SEQ ID N°18) (24 mer)
5' ACGCATGATGACAAGATTATTATC 3'
et JCA249 (SEQ ID N°19) (18 mer)
5' CTGTGGAATTCGCAATGC 3'
 ont été utilisés pour générer par PCR à partir de la matrice du plasmide pJCA001 un fragment EcoRI-bout franc de 695 pb (contenant la première partie 3' du gène FHV1 gB) = fragment E. Les oligonucléotides suivants : 
JCA221 (SEQ ID N°20)  (48 mer)
5' AAAACTGCAGCCCGGGAAGCTTACAAAAATTAGATTTGTTTCAGTATC 3'
et JCA247 (SEQ ID N°21) (36 mer)
5' GGTATGGCAAATTTCTTTCAGGGACTCGGGGATGTG 3'
 ont été utilisés pour générer par PCR à partir de la matrice du plasmide pJCA001 un fragment bout franc-PstI de 560 pb (contenant la seconde partie 3' du gène FHV-1 gB mutée au niveau du signal TTTTTNT) = fragment F.

Le fragment E a été digéré par EcoRl, puis phosphorylé. Le fragment F a été digéré par PstI, puis phosphorylé. Les fragments E et F ont alors été ligaturés ensemble avec le vecteur pIBI24 (International Biotechnologies Inc., New Haven, CT), préalablement digéré par EcoRl et PstI, pour donner le plasmide pJCA077 (contenant la cassette promoteur vaccine I3L-gène FHV-1 B).

### Construction de la cassette d'expression 42K-FHV-1 gD

Les oligonucléotides suivants : 
RG286 (SEQ ID N°22) (17 mer)
5' TTTATATTGTAATTATA 3'
et M13F (SEQ ID N°23)(17 mer)
 5' GTAAAACGACGGCCAGT 3' ont été utilisés pour générer par PCR à partir de la matrice du plasmide contenant le promoteur Entomopoxvirus AmFPV 42K (décrit dans l'exemple 21 du brevet US-A-5,505,941) un fragment EcoRI-bout franc de 130 pb (contenant le promoteur entomopox 42K) = fragment A. Les oligonucléotides suivants : 
JCA234 (SEQ ID N°24) (21 mer)
5' ATGATGACACGTCTACATTTT 3'
et JCA235 (SEQ ID N°25) (21 mer)
5' TGTTACATAACGTACTTCAGC 3'
 ont été utilisés pour générer par PCR à partir de la matrice du plasmide pFHVEcoRIM un fragment bout franc-BamHl de 185 pb (contenant la partie 5' du gène FHV-1 gD) = fragment B. Le fragment A a été digéré par EcoRl, puis phosphorylé. Le fragment B a été digéré par BamHl, puis phosphorylé. Les fragments A et B ont alors été ligaturés ensemble avec le vecteur pBluescript SK+, préalablement digéré par EcoRl et BamHl, pour donner le plasmide pJCA078.

Le plasmide pFHVEcoRIM (voir plus haut) a été digéré par BamHl et Xhol pour isoler le fragment BamHI-XhoI de 1270 pb (contenant la partie 3' du gène FHV-1 gD). Ce fragment a été alors ligaturé avec le vecteur pIBI24, préalablement digéré par BamHI et Xhol, pour donner le plasmide pJCA072. Les oligonucléotides suivants : 
JCA242 (SEQ ID N°26) (18 mer)
5' GAGGATTCGAAACGGTCC 3'
et JCA237 (SEQ ID N°27) (53 mer)
5'AATTTTCTCGAGAAGCTTGTTAACAAAAATCATTAAGGATGGTAGATTGCATG 3'
 ont été utilisés pour générer par PCR à partir de la matrice pFHVEcoRIM un fragment Xbal-Xhol de 290 pb. Ce fragment a été digéré par Xbal et Xhol = fragment C (contenant la fin du gène FHV-1 gD).

Le plasmide pJCA072 a été digéré par XbaI et Xhol pour isoler le fragment Xbal-Xhol de 3575 pb (vecteur pIBI24 + début de la partie 3' du gène FHV-1 gD) = fragment D. Les fragments C et D ont alors été ligaturés ensemble pour donner le plasmide pJCA073.

Le plasmide pJCA073 a été digéré par BamHI et Xhol pour isoler le fragment BamHI-XhoI de 960 pb (contenant la partie 3' du gène PHV-1 gD) = fragment A. Le plasmide pJCA078 a été digéré par Hpal et BamHl pour isoler le fragment HpaI-BamHI de 310 pb (contenant le promoteur 42K fusionné à la partie 5' du gène FHV-1 gD) = fragment B. Les fragments A et B ont été ligaturés ensemble avec le vecteur pBluescript SK+, préalablement digéré par EcoRV et Xhol, pour donner le plasmide pJCA080 (contenant la cassette promoteur 42K-gène FHV-1 gD).

### Construction de la cassette H6-FHV-1 gC

L'ADN génomique du virus FHV-1 (souche CO) a été digéré par EcoRI et le fragment EcoRI F d'environ 7500 pb a été cloné dans pbluescript SK+ pour donner le plasmide pFHVEcoRIF. Les oligonucléotides suivants : 
JCA274 SEQ ID N°28)(55 mer)
5'CATTATCGCGATATCCGTTAAGTTTGTATCGTAATGAGACGATATAGGATGGGAC
3'
et JCA275 (SEQ ID N°29)(18 mer)
5' ACTATTTTCAATACTGAC 3'
 ont été utilisés pour générer par PCR à partir de la matrice pFHVEcoRIF un fragment qui a été digéré par Nrul et Sall pour donner un fragment Nrul-Sall de 107 pb (contenant la partie 3' du promoteur vaccine H6 fusionnée à la partie 5' du gène FHV-1 gC) = fragment A. Les oligonucléotides suivants : 
JCA276 (SEQ ID N°30) (18 mer)

5' AAATGTGTACCACGGAC 3'

et JCA277 (SEQ ID N°31) (54 mer)

5'AAGAAGCTTCTGCAGAATTCGTTAACAAAAATCATTATAATCGCCGGGGATGAG

3'
 ont été utilisés pour générer par PCR à partir de la matrice pFHVEcoRIF un fragment qui a été digéré par EcoRV et Hindlll pour donner un fragment EcoRV-Hindlll de 370 pb (contenant la partie 3' du gène FHV-1 gC et les sites HpaI-EcoRI-PstI-HindIII) = fragment B.

Le plasmide pJCA020 (voir plus haut) a été digéré par Nrul et HindIII pour isoler le fragment HindIII-NruI (contenant la partie 5' du promoteur vaccine H6) = fragment C. Le plasmide pFHVEcoRIF a été digéré par BamHl et EccRV pour isoler le fragment BamHI-EcoRV de 580 pb (contenant la partie centrale du gène FHV-1 gC) = fragment D. Les fragments A et C ont été ligaturés ensemble avec le vecteur pbluescript SK+, préalablement digéré par Hindlll et Sall pour donner le plasmide pJCA097. Les fragments B et D ont été ligaturés ensemble avec le vecteur pBluescript SK+, préalablement digéré par BamHl et Hindlll, pour donner le plasmide pJCA099.

Le plasmide pJC097 a été digéré par Pstl et Sall pour isoler le fragment Pstl-Sall de 200 pb (contenant la cassette H6-partie 5' de gC) = fragment E. Le plasmide pFHV1EcoRIF a été digéré par BamHl et Sall pour isoler le fragment SaII-BamHI de 600 p (2ème partie centrale de FHV-1 gC) = fragment F. Les fragments E et F ont alors été ligaturés ensemble avec le vecteur pBluescript SK+, préalablement digéré par BamHl et Pstl, pour donner le plasmide pJCA098. Le plasmide pJCA098 a ensuite été digéré par EcoRl et BamHI pour isoler le fragment EcoRI-BamHI de 820 pb (contenant la cassette H6-partie 5' de FHV-1 gC) = fragment G. Le plasmide pJCA099 (voir plus haut) a été digéré par BamHl et Hindlll pour isoler le fragment BamHl-Hindlll de 960 pb (contenant la partie 3' du gène FHV-1 gC) = fragment H. Les fragments G et H ont alors été ligaturés ensemble avec le vecteur pBluescript SK+, préalablement digéré par EcoRV et Hindlll, pour donner le plasmide pJCA100 (contenant la cassette d'expression promoteur vaccine H6-gène FHV-1 gC).

Le plasmide pJCA100 a été digéré par NruI et EcoRI pour isoler le fragment NruI-EcoRI de 1650 pb contenant la partie 3' du promoteur H6 fusionnée au gène FHV-1 gC. Ce fragment a été ligaturé avec le plasmide pJCA053 (cassette VQH6-IBV M dans le vecteur pBluescript SK+), préalablement digéré par Nrul et EcoRl, pour donner le plasmide pJCA108 (contenant la cassette VQH6-gC dans pBluescript SK+). Le plasmide pJCA079 (voir plus haut) a été digéré par Smal et BamHl pour isoler le fragment BamHI-SmaI de 840 pb (contenant la cassette I3L-partie 5' du gène FHV-1 gB) = fragment A. Le plasmide pJCA079 a également été digéré par BamHl et Hindlll pour isoler le fragment BamHI-HindIII de 2155 pb (contenant la partie 3' du gène FHV-1 gB) = fragment B. Le plasmide pJCA108 (voir plus haut) a été digéré par HindIII et EcoRl pour isoler le fragment Hind(II-EcoRI de 1830 pb (contenant la cassette VQH6-FHV-1 gC) = fragment C. Le plasmide pJCA080 (voir plus haut) a été digéré par EcoRI et Xhol pour isoler le fragment EcoRI-XhoI de 1275 pb (contenant la cassette 42K-gène FHV-1 gD) = fragment D. Les fragments A, B, C et D ont alors été ligaturés ensemble avec le plasmide donneur pC6L pour donner le plasmide pJCA109.

Ce plasmide contient les cassettes d'expression H6-gène FHV-1 gC, I3L-gène FHV-1 gB et 42K-gène FHV-1 gD dans le locus C6 du virus ALVAC. La structure de ce plasmide a été vérifiée par séquençage et carte de restriction complète.

Ce plasmide est le plasmide donneur pour l'insertion des cassettes d'expression H6-gène FHV-1 gC, I3L-gène FHV-1 gB et 42K-gène FHV-1 gD dans le locus C6 du virus ALVAC.

Après linéarisaticn par Notl, le plasmide pJCA109 a été utilisé comme plasmide donneur pour la recombinaison *in vitro* (Piccini *et al.* Methods in Enzymology. 1987. 153. 545-563) avec le virus ALVAC pour générer le virus recombiné désigné vCP243.

### Exemple 8 : Adjuvant

Le carbomère utilisé dans les vaccins conformes à la présente invention est le Carbopol® 974P fabriqué par la société BF Goodrich (PM environ 3 millions).

On prépare tout d'abord une solution mère à 1,5 % P/V de Carbopol® 974P dans de l'eau distillée contenant du chlorure de sodium à 1 g/l.

Cette solution mère est ensuite utilisée pour la fabrication d'une solution de Carbopol® en eau physiologique à 4 mg/ml. La solution mère est versée dans la totalité de l'eau physiologique (ou éventuellement dans la majorité de celle-ci) en 1 fois ou éventuellement en plusieurs fois avec à chaque fois l'ajustement du pH à l'aide de NaOH (par exemple 1 N ou plus concentré) à une valeur d'environ 7,3 à 7,4.

On obtient alors une solution de Carbcpol® prête à l'emploi qui pourra être utilisée par l'utilisateur final pour reprendre un vaccin reccmbinant lyophilisé.

### Exemple 9 : Vaccination des chevaux à l'aide du vecteur canarypox recombinant vPC132 (voir exemple 6) exprimant les glycoprotéines gB, gC et gD de l'herpès virus équin de type l (EHV-1).

### 1. Protocole d'immunisation et épreuve :

20 pcneys (Welsh mountain ponies) ne présentant pas de signes sérologiques traduisant une exposition récente à EHV-1 et EHV-4 ont été répartis au hasard en 4 groupes (A à D) de 5 poneys.

Les groupes A et B ont été vaccinés avec le canarypox recombinant vCP132 exprimant les glycoprotéines gB, gC et gD de la souche Kentucky D de EHV-1. Le vaccin a été repris dans de l'eau stérile (groupe A) ou dans une solution de carbomère 4 mg/ml (groupe B) selon l'exemple 8.

Le groupe C a été vacciné avec un vaccin EHV entier inactivé du commerce contenant, sous un volume de dose de 1,5 ml, des valences EHV-1 et EHV-4 inactivées et 6 mg de carbomère.

Le groupe D est le groupe témoin dans lequel les animaux ont été vaccinés avec un virus canarypox recombinant vCP1502 exprimant la glycoprotéine HA du virus Influenza A/equi-1/Prague56 (voir exemple 3) repris dans du carbomère dans les mêmes conditions que pour le groupe B.

Les vaccins sont décrits en détail dans le tableau I :

| Groupes | Vaccins | Antigènes | Diluant / Adjuvant | Dose (1 ml) |
|---|---|---|---|---|
| A | vCP132 | EHV-1 | Eau stérile | 10^{8.0} TCID₅₀ |
| B | vCP132 | EHV-1 | Carbopol® 974P | 10^{8.0}TCID₅₀, |
| C | vaccin du commerce | EHV-1 EHV-4 | Carbopol® | 10^{7.3} TCID50 avant inactivation EHV-1 10^{7.3} TCID50 avant inactivation EHV-4 |
| D | vCP1502 | HA-Prague 56 | Carbopol® 974P | 10^{a}TCID₅₀ |

Chaque animal a reçu 1 dose du vaccin correspondant à J0 et J35 par injection intramusculaire profonde dans le cou.

A J 56, les poneys ont été éprouvés par instillation intra-nasale de 10⁵ TCID₅₀ de la souche Ab4/8 de EHV-1.

### 2. Tests sérologiques :

On a réalisé des tests de neutralisation SN selon la technique décrite dans Thompson et al., Equine Vet. J., 8, 58-65, 1976. Le virus EHV-1 (RACH) a été utilisé comme antigène.

Les titres SN sont exprimés comme l'inerse de la dilution de sérum donnant 50 % de neutralisation (log₁₀).

### 3. Suivi virologique :

L'expression de virus a été suivie quotidiennement sur 10 jours par écouvillonnages nasopharyngés que l'on a récolté dans du milieu de transport de virus. Les extraits d'écouvillonnage ont été titrés sur des cellules de reins de lapins RK13 dans des plaques de microtitration. Les titres ont été calculés en utilisant la formule de Karber exprimée en log₁₀ TCID₅₀ pour 1 ml.

### 4. Résultats :

Aucune réaction locale ou systémique significative n'a été notée suite à ces vaccinations.

Les réponses en anticorps séroneutralisants SN (log 10 de la dilution entraînant 50 % de neutralisation) moyennes sont :

| Groupe | titre à J.0 | titre à J.56 (avant épreuve) |
|---|---|---|
| A | 1, 69 ± 0, 49 | 1,93 ± 0,15 |
| B | 1,69 ± 0,47 | 2,61 ± 0,42 |
| C | 1,19 ± 0,30 | 2,47 ± 0,32 |
| D | 1,57 ± 0,45 | 1,55 ± 0,37 |

On observe avec le vaccin vCP132 en carbomère une augmentation significative du titre en anticorps.

La totalité des 5 poneys témoins excrète du virus par le nasopharynx. L'excrétion virale chez ces poneys non vaccinés a continué pendant une moyenne de 5 jours, avec un maximum d'excrétion virale à 4 jours post-infection.

Tous les poneys des groupes A, C, D excrètent du virus après épreuve. Au contraire seuls deux poneys sur les 5 poneys du groupe B vaccinés selon l'invention excrètent du virus. En outre, la quantité de virus excrétée dans le groupe B est significativement inférieure à la quantité excrétée par les autres groupes y compris le groupe C. De même. la durée d'excrétion chez les animaux du groupe B est beaucoup plus courte que dans les autres groupes.

On peut se reporter à la figure 1 et aux valeurs d'aire sous la courbes données ci-après, qui font apparaître très clairement la quasi-absence d'excrétion virale chez les poneys vaccinés par le vCP132 en présence de carbomère. Le résultat est très significatif si on le compare en particulier au vaccin commercial. On constate une réduction significative de l'excrétion virale chez les animaux du groupe B par rapport aux témoins, alors qu'il n'est pas constaté de différence significative entre les animaux du groupe C et les témoins. Cette réduction d'un degré remarquable et inattendu de l'excrétion de virus est particulièrement intéressante pour ses implications très favorables sur la limitation de la transmission du virus de cheval à cheval.

### Virus total par poney (aire sous la courbe) :

A: 17,1
B : 3,0
C : 9,7
D : 16,3

### Exemple 10 : vaccination de chevaux à l'aide du vecteur canarypox vCP1533 (voir exemple 5) recombinant exprimant la glycoprotéine HA du virus Influenza A/equi-2/Newmarket/2/93 en présence de carbomère :

### 1. Protocole d'immunisation et épreuve:

20 poneys (Welsch mountain ponies), de 7 à 8 mois d'âge, ne présentant pas d'anticorps détectables contre les virus H3N8 et H7N7, mesurés par le test SRH (pour single radial hemolysis ou hémolyse radiale simple), ont été utilisés dans cette étude. Le statut négatif des animaux permet d'étudier dans les meilleures conditions l'efficacité des différents vaccins en termes de réponse humorale. Les poneys ont été répartis au hasard en 4 groupes (A à D) de 5 à 6 poneys.

Les poneys du groupe A ont été vaccinés à l'aide d'un canarypox recombinant (vCP1533) exprimant la glycoprotéine HA du virus influenza A/equi-2/Newmarket/2/93. Ce vaccin a été repris dans une solution contenant 4 mg/ml de carbomère, carbopol® 974P.

Le groupe B a été vacciné avec un vaccin commercial contenant, sous un volume de dose de 1,5 ml, un mélange de 3 souches inactivées d'influenza à savoir Prague/56, Suffolk/89 et Miami/63, de l'anatoxine tétanique, ainsi que du carbomère (4 mg) et de l'hydroxyde d'aluminium (2,2 mg) comme adjuvants.

Le groupe C a été vacciné à l'aide d'un vaccin C comprenant 2 valences inactivées Influenza à savoir Prague/56, Newmarket/2/93, ainsi que de l'anatoxine tétanique, dans de l'hydroxyde d'aluminium.

Le groupe D a été vacciné à l'aide d'un vecteur canarypox recombinant vCP132 vu ci-dessus et reconstitué avec une solution contenant 4 mg/ml de carbomère 974P. Ce dernier groupe a servi de témoin pour l'épreuve.
Les vaccins sont décrits en détails dans le tableau Il :

| Groupes | Vaccins | Antigènes | Diluant / Adjuvant | Dose (1 ml) |
|---|---|---|---|---|
| A | vCP1533 | HA-Newmarket/2/93 | Carbopol® 974P | 10^{7.7}TCID₅₀ |
| B | Vaccin du commerce | Prague/56: Suffolk/89 Miami/63 ; anatoxine tétanique | Carbopoi® Al(OH)₃ | 15 µgHA de chaque souche |
| C | Vaccin C | Prague/56; Newmarket/2/93 anatoxine tétanique | AI(OH)₃ | 15 µg HA de chaque souche |
| D | vCP132 | gB, gC, gD - EHV-1 | Carbopol® 974P | 10^{8.0}TCID₅₀ |

2 doses de 1 ml de chaque vaccin ont été administrées à chaque animal à 5 semaines d'intervalle par injection intramusculaire profonde dans le cou.

2 semaines après la deuxième vaccination, chaque poney a été infecté par exposition à un aérosol provenant d'environ 20 ml de fluide allantoïdien pour un total de 10^{7.3} EiD₅₀ de virus influenza A-equi-2/Sussex/89, en utilisant un nébulisateur de modèle ULTRA 2000 (De Villbiss, Somerset PA) comme décrit par Mumford et al, Equine Vet, J., 22 : 93-98, 1990.

### 2. Test sérologique :

Des échantillons de sang total ont été collectés aux jours suivants : 0 (le même jour que et avant la première vaccination), 7, 14, 35 (le même jour que et avant la deuxième vaccination), 49 (le même jour que et avant l'épreuve), 56 et 63.

Le sérum a été préparé et stocké et conservé par congélation à - 20° C jusqu'à son utilisation. Tous les sérums ont été testés pour la présence d'anticorps SRH vis-à-vis de Influenza A/equi-1/Prague/56 et Influenza A/equi-2/Newmarket/2/93 comme décrit par Wood et al. (J. Hyg.. 90 : 371-384, 1983).

Les diamètres des zones d'hémolyse ont été mesurés dans deux directions à angle droit en utilisant un lecteur automatique. La superficie des zones a été calculée et une augmentation de 50 % a été considérée comme significative. Les titres ont été exprimés en mm² d'hémolyse.

### 3. Suivi virologigue.

L'excrétion virale a été suivie quotidiennement sur 10 jours en récoltant des écouvillonnages nasopharyngés dans un milieu de transport de virus. L'exsudat de chaque écouvillonnage a été dilué par dilutions de raison 10 dans du PBS à pH7,2 et 0,1 ml de chaque dilution a été inoculé dans l'espace allantoïdien d'oeufs embryonnés de 10 jours d'âge. Le titre viral (EID₅₀ /ml) dans les extraits des écouvillonnages a été calculé à partir de l'activité hémagglutinante dans des liquides allantoïdiens récoltés après 72 heures d'incubation des oeufs à 34°C.

### 4. Résultats.

Il n'a pas été constaté de réaction locale ou systémique significative à la suite de la première vaccination à l'exception d'un cheval du groupe B.

A noter que les souches Suffolk et Newmarket sont semblables(Daly et al., J. Gen. Virol. 1996. 77. 661-671), ce qui rend parfaitement valable la comparaison avec le vaccin commercial dans les conditions de l'essai.

Aucun des poneys n'avait d'anticorps SRH détectable contre Influenza A/equi-2/Newmarket/2/93 ou Influenza A/equi-1/Prague/56 au début de l'étude. Les résultats sérologiques à 1 semaine après ia première vaccination ont montré qu'aucun des poneys n'avait été préalablement infecté par Influenza (pas d'effet rappel observable).

2 semaines après la première vaccination, aucun des poneys n'a développé de réponse en anticorps détectable contre Influenza A/equi-1/Prague/56. En outre, il n'y a pas eu d'anticorps SRH détectable contre Influenza A/equi-2/Newmarket/93 chez 6 animaux sur 6 vaccinés avec vaccin C, chez 4 animaux sur 5 vaccinés avec le vaccin du commerce B et dans le groupe témoin D. Au contraire un titre en anticorps SRH très fort a été constaté chez l'ensemble des 5 poneys vaccinés avec le canarypox en présence de l'adjuvant carbomère : moyenne 155,4 ± 32,9. Le tableau III suivant présente les résultats obtenus animal par animal en ce qui concerne les titres en anticorps SRH.

**Tableau III**

| Résultats SRH (mm²) | | | | | |
|---|---|---|---|---|---|
| Poneys | Groupe | PRAGUE (J0, J7, J14) | NEWMARKET | | |
| | | | J0 | J7 | J14 |
| M26 | A | 0 | 0 | 0 | 158.0 |
| M27 | A | 0 | 0 | 0 | 104.2 |
| M28 | A | 0 | 0 | 0 | 160.3 |
| M29 | A | 0 | 0 | 0 | 196.4 |
| M30 | A | 0 | 0 | 0 | 158.0 |
| M31 | B | 0 | 0 | 0 | 0 |
| M32 | B | 0 | 0 | 0 | 0 |
| M33 | B | 0 | 0 | 0 | 0 |
| M34 | B | 0 | 0 | 0 | 0 |
| M35 | B | 0 | 0 | 0 | 81.2 |
| M36 | C | 0 | 0 | 0 | 0 |
| M37 | C | 0 | 0 | 0 | trace |
| M38 | C | 0 | 0 | 0 | 0 |
| M39 | C | 0 | 0 | 0 | 0 |
| M40 | C | 0 | 0 | 0 | trace |
| M41 | C | 0 | 0 | 0 | 0 |
| M42 | D | 0 | 0 | 0 | 0 |
| M43 | D | 0 | 0 | 0 | 0 |
| M44 | D | 0 | 0 | 0 | 0 |
| M45 | D | 0 | 0 | 0 | 0 |
| M46 | D | 0 | 0 | 0 | 0 |

Le vaccin selon l'invention conduit à l'apparition d'un haut titre en anticorps dès 14 jours après la première vaccination aiors que, globalement, pour les vaccins B et C, la première vaccination n'entraîne pas à cette date l'apparition d'anticorps à des niveaux détectables. L'obtention aussi précoce d'un tel titre est un résultat remarquable et inattendu, jamais constaté auparavant.

### Exemple 11 : Vaccination des chevaux à l'aide du vecteur canarypox vCP1502 (voir exemple 3) recombinant exprimant la glycoprotéine HA du virus influenza A/equi-1/Prague 56 en présence de carbomère

Les témoins de l'exemple 1, vaccinés par vCP1502, ont aussi été suivis sur le plan sérologique.

Le tableau IV suivant montre les titres IHA (inhibition de l'hémagglutination) obtenus chez les animaux immunisés avec 10^{a} pfu de vCP1502 avec Carbopol® 974P à 0 (1ère injection V1) et 35 (2ème injection V2) jours.

**Tableau IV**

| | **titres anti-H7N7 IHA** | | | | |
|---|---|---|---|---|---|
| Poneys | jour 0 (V1 ) | jour 7 | jour 14 | jour 35 (V2) | jour 56 |
| RM16 | 0 | 0 | 128 | 64 | **128** |
| RM17 | 0 | 0 | 32 | 128 | **256** |
| RM18 | 0 | 0 | 16 | 64 | **512** |
| RM19 | 0 | 0 | 32 | 32 | **256** |
| RM20 | 0 | 0 | 128 | 64 | **128** |

Comme dans l'exemple précédent, on constate que l'injection d'un canarypox -EIV (exprimant le gène HA du virus A équi-1/Prague) mélangé à du carbomère permet l'obtention de titres IHA spécifiques élevés dès J14 après une vaccination. De manière remarquable, ces titres élevés sont encore augmentés de manière significative après rappel pour atteindre un titre moyen très élevé, d'un niveau jamais constaté auparavant pour l'antigène HA de virus EIV H7N7 sur chevaux non primo-stimulés.

### Exemple 12 : application chez le chat

Le virus recombiné testé est un virus canarypox recombiné exprimant les gènes gB, gC et gD de l'herpèsvirus féiin (Feline Herpesvirus = FHV). Ce virus recombiné est identifié vCP243 (voir exemple 7)

Le protocole de vaccination/épreuve dans le modèle FHV est le suivant.

| Groupe | Nombre de chats | Vaccin | Diluant/ Adjuvant | Dose |
|---|---|---|---|---|
| A | 6 | vCP243 | eau | 10^{7.5} pfu |
| B | 6 | vCP243 | Carbopol® 974P | 10^{7,5}pfu |
| C | 6 | CORIFELIN® | - | 1 dose commerciale |
| D (témoins) | 6 | - | - | - |

Les chats sont vaccinés à J0 et J28 par voie sous-cutanée.

Le vaccin CORIFELIN® est un vaccin FHV sous-unités commercialisé par Mérial, Lyon, France, comprenant au moins 200 unités IDR de fractions virales de FHV, 25 µg d'antigène purifié de calicivirus félin, 0,1 mg du thiomersal et de l'excipient huileux QSP 1 ml.

L'épreuve est effectuée à J49, par voie oronasale d'une souche d'épreuve FHV.

Le suivi clinique est effectué pendant 14 jours après épreuve en notant les signes cliniques (notation des signes cliniques selon les règles de la Pharmacopée Européenne).

La protection est appréciée après d'épreuve sur les critères suivants :
- scores cliniques moyens de chaque groupe, comparés entre eux et au score clinique moyen du groupe témoin
- niveau d'excrétion virale FHV après épreuve (mesure de la charge virale dans des écouvillonnages pharyngés réalisés quotidiennement de J0 à J10 après épreuve)
- titres anticorps neutralisants le virus FHV sur prises de sang à J0, J28, J49, J63.

Pour tous ces critères, les taux moyens de chaque groupe sont également comparés entre eux et au taux moyen du groupe témoin.

### Exemple 13 : application chez le chien

Le virus recombiné testé est un virus canarypox recombiné exprimant les gènes HA et F du virus de la maladie de Carré (Canine Distemper Virus, CDV). Ce virus recombiné est identifié **vCP258** (voir exemple 2).

Le protocole de vaccination/épreuve dans le modèle CDV est le suivant.

| Groupe | Nombre de chiens | Vaccin | Diluant / Adjuvant | Dose |
|---|---|---|---|---|
| A | 6 | vCP258 | eau | 10^{7.0}pfu |
| B | 6 | vCP258 | carbopol® 974P | 10^{7.0}pfu |
| C | 6 | EURICAN® | - | 1 dose commerciale |
| D (témoins) | 6 | - | - | - |

Les chiens sont vaccinés à J0 et J28 par voie sous-cutanée.

Le vaccin EURICAN® (CHPP12) est un vaccin vivant commercialisé par Merial, Lyon, France. Une dose commerciale contient au minimum 10⁴ pfu de la souche vaccinale CDV Onderstepoort

L'épreuve est effectuée à J56, par administration intracranienne d'une dilution au 1/10ème de la souche d'épreuve CDV "Snyder-Hill" (lot préparé et fourni par l'USDA). Le suivi clinique est effectué pendant 21 jours après épreuve en notant les signes cliniques (notation des signes cliniques selon les règles de la Pharmacopée Européenne).

La protection est apprécié après épreuve sur les cr tères suivants :
- scores cliniques moyens de chaque groupe, comparés entre eux et au score clinique moyen du groupe témoin
- niveau de virémie CDV après épreuve (mesure de la charge virale dans les lymphocytes à J56, J61, J63, J66, J70, J77)
- titres anticorps neutralisants le virus CDV à J0, J14, J28, J42, J56, J63, J77.

Pour tous ces critères, les taux moyens de chaque groupe sont également comparés entre eux et au taux moyen du groupe témoin.

## Revendications

1. Vaccin vivant recombinant comprenant un vecteur viral incorporant et exprimant *in vivo* une séquence nucléotidique hétérologue sélectionnée parmi un gène d'un herpès virus animal et un gène d'un virus influenza, et au moins un composé adjuvant choisi parmi les polymères de l'acide acrylique ou méthacrylique et les copolymères d'anhydride maléique et de dérivé alcényle.

2. Vaccin selon la revendication 1, **caractérisé en ce qu'**il comprend comme composé adjuvant un polymère de l'acide acrylique ou méthacrylique réticulé par un éther polyalcénylique de sucre ou de polyalcool.

3. Vaccin selon la revendication 2, **caractérisé en ce que** le polymère est réticulé par un allyl saccharose ou par de l'allylpentaérythritol.

4. Vaccin selon la revendication 1, **caractérisé en ce qu'**il comprend comme composé adjuvant un copolymère d'anhydride maléique et d'éthylène linéaire ou réticulé, par exemple réticulé par du divinyléther.

5. Vaccin selon la revendication 1, **caractérisé en ce que**, comme composé adjuvant, il comprend un carbomer.

6. Vaccin selon l'une quelconque des revendications 1 à 5, **caractérisé en ce que** le composé adjuvant est présent dans le vaccin à raison de 0,01 % à 2 % PN.

7. Vaccin selon la revendication 6, **caractérisé en ce que** la concentration est de 0,06 à 1 % en PN, de préférence de 0,1 à 0,6 % en P/V.

8. Vaccin selon l'une des revendications 1 à 7, **caractérisé en ce qu'**il comprend un vecteur incorporant et exprimant au moins un gène d'un herpès virus animal.

9. Vaccin selon la revendication 8, **caractérisé en ce que** le gène provient d'un herpès virus choisi dans le groupe consistant en herpès virus équin EHV-1 et/ou EHV-4, herpès virus félin FHV, herpès virus canin CHV, herpès virus aviaire ILTV ou Marek, herpès virus bovin BHV et herpès virus porcin PRV.

10. Vaccin selon l'une quelconque des revendications 1 à 7, **caractérisé en ce qu'**il comprend au moins un vecteur incorporant et exprimant au moins un gène d'un virus influenza.

11. Vaccin selon la revendication 10, **caractérisé en ce que** le vecteur exprime un gène d'un virus influenza choisi dans le groupe consistant en virus grippe équine, virus grippe aviaire et virus grippe porcine.

12. Vaccin selon l'une quelconque des revendications 1 à 7, destiné à la vaccination d'un animal de l'espèce équine et comprenant un vecteur viral incorporant un gène de l'herpèsvirus équin ou un gène d'un virus influenza.

13. Vaccin selon la revendication 12, **caractérisé en ce qu'**il comprend un gène de l'herpèsvirus équin EHV-1 et/ou EHV-4.

14. Vaccin selon la revendication 12, **caractérisé en ce qu'**il comprend un gène d'un virus influenza équin.

15. Vaccin selon l'une quelconque des revendications 1 à 14, **caractérisé en ce que** le vecteur viral est choisi dans le groupe consistant en poxvirus, adénovirus, herpès virus.

16. Vaccin selon la revendication 15, **caractérisé en ce que** le poxvirus est choisi dans le groupe consistant en virus de la vaccine, fowlpox, canarypox, pigeonpox, swinepox.

17. Vaccin selon la revendication 16, **caractérisé en ce que** le virus de la vaccine est le NYVAC.

18. Vaccin selon la revendication 13, **caractérisé en ce qu'**il comprend un vecteur canarypox incorporant et exprimant les gènes gB, gC et gD du virus herpès équin EHV-1 et/ou EHV-4.

19. Vaccin selon la revendication 14, **caractérisé en ce qu'**il comprend deux ou trois vecteurs canarypox comprenant chacun un gène HA du virus influenza équin, chaque gène HA provenant d'une souche différente.

20. Vaccin selon la revendication 14, **caractérisé en ce qu'**il comprend un vecteur canarypox comprenant deux ou trois gènes HA de souches différentes de virus influenza équin.

21. Vaccin selon revendication 19 ou 20, **caractérisé en ce qu'**il comprend deux ou trois gènes HA provenant des souches Prague, Kentucky et/ou Newmarket.

22. Vaccin selon l'une des revendications 16, 18-21, **caractérisé en ce que** le vecteur canarypox est un vecteur ALVAC.

23. Vaccin selon l'une quelconque des revendications 1 à 7, destiné à la vaccination d'un animal d'une espèce aviaire contre le virus influenza et comprenant un vecteur viral incorporant un gène d'un virus influenza.

24. Ensemble de vaccination comprenant, conditionnés séparément, d'une part, sous forme lyophilisée, un vaccin vivant recombinant comprenant un vecteur viral incorporant et exprimant *in vivo* une séquence nucléotidique hétérologue sélectionnée parmi un gène d'un herpès virus animal et un gène d'un virus influenza, de préférence tel que défini dans l'une quelconque des revendications 8 à 23, et d'autre part, une solution d'au moins un composé adjuvant choisi parmi les polymères de l'acide acrylique ou méthacrylique et les copolymères d'anhydride maléique et de dérivé alcényle, tel que défini dans l'une quelconque des revendications 1 à 7, la solution d'adjuvant étant destinée à la reprise du vaccin lyophilisé.

25. Utilisation d'un composé choisi parmi les polymères de l'acide acrylique ou méthacrylique et les copolymères d'anhydride maléique et de dérivé alcényle, tels que définis dans l'une quelconque des revendications 1 à 7, et d'un vecteur viral comprenant et exprimant *in vivo* la séquence nucléotidique d'un gène d'un herpès virus animal, pour la production d'un vaccin vivant recombiné adjuvé destiné à protéger un animal vis-à-vis de de la maladie causée par cet herpès virus.

26. Utilisation selon la revendication 25, **caractérisée en ce que** le gène est un gène d'un herpès virus équin EHV-1 et/ou EHV-4, pour la production d'un vaccin vivant recombiné adjuvé destiné à protéger un animal de l'espèce équine vis-à-vis de la maladie causée par cet herpès virus.

27. Utilisation d'un composé choisi parmi les polymères de l'acide acrylique ou méthacrylique et les copolymères d'anhydride maléique et de dérivé alcényle, tels que définis dans l'une quelconque des revendications 1 à 7, et d'un vecteur viral incorporant et exprimant *in vivo* la séquence nucléotidique d'un gène d'un virus influenza, pour la production d'un vaccin vivant recombiné destiné à protéger un animal contre la grippe.

28. Utilisation selon la revendication 27, **caractérisée en ce que** le gène est un gène d'un virus influenza équin, pour la production d'un vaccin vivant recombiné adjuvé destiné à protéger un animal de l'espèce équine contre la grippe équine.

29. Utilisation d'un composé choisi parmi les polymères de l'acide acrylique ou méthacrylique et les copolymères d'anydride maléique et de dérivé alcényle, tels que définis dans l'une quelconque des revendications 1 à 7, et d'un vecteur viral incorporant et exprimant *in vivo* la séquence nucléotidique d'un gène d'un herpès virus équin, pour la production d'un vaccin vivant recombiné adjuvé permettant d'induire chez un animal de l'espèce équine une diminution significative de l'excrétion virale relative à l'herpès virus équin.

30. Utilisation d'un composé choisi parmi les polymères de l'acide acrylique ou méthacrylique et les copolymères d'anydride maléique et de dérivé alcényle, tels que définis dans l'une quelconque des revendications 1 à 7, et d'un vecteur viral incorporant et exprimant *in vivo* la séquence nucléotidique d'un gène d'un virus influenza, pour la production d'un vaccin vivant recombiné adjuvé permettant d'induire chez un animal de l'espèce équine une diminution significative de l'excrétion virale relative au virus influenza.

31. Utilisation d'un composé choisi parmi les polymères de l'acide acrylique ou méthacrylique et les copolymères d'anhydride maléique et de dérivé alcényle, tels que définis dans l'une quelconque des revendications 1 à 7, et d'un vecteur viral incorporant et exprimant *in vivo* la séquence nucléotidique d'un gène d'un virus influenza, pour la production d'un vaccin vivant recombiné adjuvé permettant d'induire chez un animal de l'espèce équine la production précoce d'anticorps contre le virus influenza.

32. Utilisation selon la revendication 31, **caractérisée en ce qu'**il s'agit d'une production d'anticorps observée après une seule administration.

33. Utilisation d'un composé choisi parmi les polymères de l'acide acrylique ou méthacrylique et les copolymères d'anydride maléique et de dérivé alcényle, tels que définis dans l'une quelconque des revendications 1 à 7, et d'un vecteur viral incorporant et exprimant *in vivo* la séquence nucléotidique d'un gène d'un virus influenza, pour la production d'un vaccin vivant recombiné adjuvé destiné à protéger un animal d'une espèce aviaire contre la grippe aviaire.

34. Utilisation selon l'une quelconque des revendications 25 à 33, **caractérisée en ce que**, comme adjuvant, l'on utilise un carbomer.

35. Utilisation selon l'une quelconque des revendications 25 à 34, **caractérisée en ce que** le vecteur viral est un poxvirus, de préférence choisi parmi virus de la vaccine, fowlpox, canarypox, pigeonpox et swinepox.

36. Utilisation selon la revendication 28, 30, 31 ou 32, **caractérisée en ce que** le vecteur viral est un canarypox comprenant un gène HA du virus influenza.

37. Utilisation selon la revendication 36, **caractérisée en ce que** l'on utilise deux ou trois vecteurs canarypox comprenant un gène HA du virus influenza équin, chaque gène HA provenant d'une souche différente.

38. Utilisation selon la revendication 37, **caractérisée en ce qu'**il s'agit des souches Prague, Kentucky et Newmarket.

39. Utilisation selon la revendication 26 ou 29, **caractérisée en ce que** le vecteur viral est un canarypox comprenant les gènes gB, gC et gD de EHV-1 et/ou EHV-4.

## Claims

1. Live recombinant vaccine comprising a viral vector Incorporating and expressing *in vivo* a heterologous nucleotide sequence selected from a gene of an animal herpes virus and a gene of an influenza virus, and at least one adjuvant compound chosen from the acrylic or methacrylic acid polymers and the maleic anhydride and alkenyl derivative copolymers.

2. Vaccine according to claim 1, **characterized in that** it comprises as adjuvant compound, an acrylic or methacrylic acid polymer cross-linked by a sugar or polyalcohol polyalkenyl ether.

3. Vaccine according to claim 2, **characterized in that** the polymer is cross-linked by a saccharose allyl or by allylpentaerythritol.

4. Vaccine according to claim 1, **characterized in that** it comprises as adjuvant compound a linear or cross-linked ethylene-maleic anhydride copolymer, for example cross-linked by divinyl ether.

5. Vaccine according to claim 1, **characterized in that**, as adjuvant compound, it comprises a carbomer.

6. Vaccine according to any one of claims 1 to 5, **characterized in that** the adjuvant compound is present in the vaccine at a rate of 0.01% to 2% w/v.

7. Vaccine according to claim 6, **characterized in that** the concentration is 0.06% to 1% w/v, preferably 0.1 % to 0.6% w/v.

8. Vaccine according to one of claims 1 to 7, **characterized In that** it comprises a vector incorporating and expressing at least one gene of an animal herpes virus.

9. Vaccine according to claim 8, **characterized In that** the gene originates from a herpes virus chosen from the group consisting of equine herpes virus EHV-1 and/or EHV-4, feline herpes virus FHV, canine herpes virus CHV, avian herpes virus ILTV or Marek's disease virus, bovine herpes virus BHV or porcine herpes virus PHV.

10. Vaccine according to any one of claims 1 to 7, **characterized In that** it comprises at least one vector Incorporating and expressing at least one gene of an Influenza virus.

11. Vaccine according to claim 10, **characterized in that** the vector expresses a gene of an influenza virus chosen from the group consisting of equine influenza virus, avian influenza virus and porcine Influenza virus.

12. Vaccine according to any one of claims 1 to 7, Intended for the vaccination of an animal of the equine species and comprising a viral vector incorporating a gene of the equine herpes virus or a gene of an influenza virus,

13. Vaccine according to claim 12, **characterized in that** it comprises a gene of the equine herpes virus EHV-1 and/or EHV-4.

14. Vaccine according to claim 12, **characterized in that** it comprises a gene of an equine influenza virus.

15. Vaccine according to any one of claims 1 to 14, **characterized in that** the viral vector is chosen from the group consisting of a pox virus, adenovirus, herpes virus.

16. Vaccine according to claim 15, **characterized in that** the pox virus is chosen from the group consisting of vaccine virus, fowl pox, canary pox, pigeon pox, swine pox.

17. Vaccine according to claim 16, **characterized in that** the vaccine virus is NYVAC.

18. Vaccine according to claim 13, **characterized In that** it comprises a canary pox vector incorporating and expressing the genes gB, gC and gD of the equine herpes virus EHV-1 and/or EHV-4.

19. Vaccine according to claim 14, **characterized in that** it comprises two or three canary pox vectors each comprising an HA gene of the equine Influenza virus, each HA gene originating from a different strain.

20. Vaccine according to claim 14, **characterized in that** it comprises a canary pox vector comprising two or three HA genes of different strains of equine Influenza virus.

21. Vaccine according to claim 19 or 20, **characterized in that** It comprises two or three HA genes originating from the Prague, Kentucky and/or Newmarket strains.

22. Vaccine according to one of claims 16, 18-21, **characterized in that** the canary pox vector is an ALVAC vector.

23. Vaccine according to any one of claims 1 to 7, intended for the vaccination of an animal of an avian species against the influenza virus and comprising a viral vector incorporating a gene of an influenza virus.

24. Vaccination pack comprising, packaged separately, on the one hand, in lyophilized form a live recombinant vaccine comprising a viral vector incorporating and expressing *in vivo* a heterologous nucleotide sequence selected from a gene of an animal herpes virus and a gene of an influenza virus, preferably as defined in any one of claims 8 to 23, and on the other hand, a solution of at least one adjuvant compound chosen from the acrylic or methacrylic acid polymers and the maleic anhydride and alkenyl derivative copolymers, as defined in any one of claims 1 to 7, the adjuvant solution being intended for taking up the lyophilized vaccine.

25. Use of a compound chosen from the acrylic or methacrylic acid polymers and the maleic anhydrido and alkenyl derivative copolymers, as defined in any one of claims 1 to 7 and a viral vector comprising and expressing *in vivo* the nucleotide sequence of a gene of an animal herpes virus, for the production of an adsorbed live recombinant vaccine intended to protect an animal from the disease caused by this herpes virus.

26. Use according to claim 25, **characterized in that** the gene is a gene of an equine herpes virus EHV-1 and/or EHV-4, for the production of an adsorbed live recombinant vaccine intended to protect an animal of the equine species from the disease caused by this herpes virus.

27. Use of a compound chosen from the acrylic or methacrylic acid polymers and the maleic anhydride and alkenyl derivative copolymers, as defined in any one of claims 1 to 7 and a viral vector incorporating and expressing *in vivo* the nucleotide sequence of a gene of an influenza virus, for the production of a live recombinant vaccine intended to protect an animal from influenza.

28. Use according to claim 27, **characterized in that** the gene is a gene of an equine influenza virus, for the production of an adsorbed live recombinant vaccine intended to protect an animal of the equine species from equine influenza.

29. Use of a compound chosen from the acrylic or methacrylic acid polymers and the malein anhydride and alkenyl derivative copolymers, as defined in any one of claims 1 to 7 and a viral vector Incorporating and expressing *in vivo* the nucleotide sequence of a gene of an equine herpes virus, for the production of an adsorbed live recombinant vaccine making it possible to induce in an animal of the equine species a significant reduction in viral excretion relative to the equine herpes virus.

30. Use of a compound chosen from the acrylic or methacrylic acid polymers and the maleic anhydride and alkenyl derivative copolymers, as defined in any one of claims 1 to 7 and a viral vector incorporating and expressing *in vivo* the nucleotide sequence of a gene of an Influenza virus, for the production of an adsorbed live recombinant vaccine making it possible to induce In an animal of the equine species a significant reduction in the viral excretion relative to the Influenza virus.

31. Use of a compound chosen from the acrylic or methacrylic acid polymers and the maleic anhydride and alkenyl derivative copolymers, as defined in any one of claims 1 to 7, and a viral vector incorporating and expressing *in vivo* the nucleotide sequence of a gene of an influenza virus, for the production of an adsorbed live recombinant vaccine making it possible to induce in an animal of the equine species the early production of antibodies against the influenza virus.

32. Use according to claim 31, **characterized in that** a production of antibodies is observed after a single administration.

33. Use of a compound chosen from the acrylic or methacrylic acid polymers and the maleic anhydride and alkenyl derivative copolymers, as defined in any one of claims 1 to 7 and a viral vector incorporating and expressing *in vivo* the nucleotide sequence of a gene of an influenza virus, for the production of an adsorbed live recombinant vaccine intended to protect an animal of an avian species against avian influenza.

34. Use according to any one of claims 25 to 33, **characterized in that**, as adjuvant, a carbomer is used.

35. Use according to any one of claims 25 to 34, **characterized in that** the viral vector is a pox virus, preferably chosen from vaccine virus, fowl pox, canary pox, pigeon pox and swine pox.

36. Use according to claim 28, 30, 31 or 32, **characterized in that** the viral vector is a canary pox comprising an HA gene of the influenza virus.

37. Use according to claim 36, **characterized in that** two or three canary pox vectors comprising an HA gene of the equine influenza virus are used, each HA gene originating from a different strain.

38. Use according to claim 37, **characterized in that** the strains are Prague, Kentucky and Newmarket.

39. Use according to claim 26 or 29, **characterized in that** the viral vector is a canary pox comprising the genes gB, gC and gD of EHV-1 and/or EHV-4.

## Patentansprüche

1. Rekombinanter Lebendimpfstoff, umfassend einen viralen Vektor, welcher *in vivo* eine heterofoge Nucleotidsequenz enthält und exprimiert, ausgewählt aus einem Gen eines tierischen Herpesvirus und einem Gen eines Influenzavirus und mindestens einem Adjuvans, ausgewählt aus den Polymeren der Acrylsäure oder Methacrylsäure und Copolymeren von Maleinsäureanhydrid und Alkenyl-Derivaten.

2. Impfstoff nach Anspruch 1, dadurch charakterisiert, dass er als Adjuvans ein Polymer der Acrylsäure oder Methacrylsäure umfasst, welches vemetzt ist mit einem Polyalkenylether eines Zuckers oder Polyalkohols.

3. Impfstoff nach Anspruch 2, dadurch charakterisiert, dass das Polymer mit einer Allylsacharose oder mit Allylpentaerythrit vemetzt ist.

4. Impfstoff nach Anspruch 1, dadurch charakterisiert, dass er als Adjuvans ein Copolymer von Maleinsäureanhydrid und einem linearen oder vemetzten Ethylen, z. B. vernetzt mit dem Divinylether, umfasst.

5. Impfstoff nach Anspruch 1, dadurch charakterisiert, dass er als Adjuvans ein Carbomer umfasst.

6. Jmpfstoff nach einem der Ansprüche 1 bis 5, dadurch charakterisiert, dass das Adjuvans im Bereich von 0,01 bis 2 Volumenprozent in dem Impfstoff vorhanden ist.

7. Impfstoff nach Anspruch 6, dadurch charakterisiert, dass die Konzentration im Bereich von 0,06 bis 1 Volumenprozent ist, vorzugsweise Zwischen 0,1 und 0,6 Volumenprozent.

8. Impfstoff nach einem der Ansprüche 1 bis 7, dadurch charakterisiert, dass er einen Vektor umfasst, welcher mindestens ein Gen eines tierischen Herpesvirus enthält und exprimiert.

9. Impfstoff nach Anspruch 8, dadurch charakterisiert, dass das Gen aus einem Herpesvirus stammt, ausgewählt aus der Gruppe bestehend aus Pferdeherpesvirus EHV-1 und/oder EHV-4, Katzenherpesvirus FHV, Hundeherpesvirus CHV, Vogelherpesvirus ILTV oder Marek, Rinderherpesvirus BHV und Schweineherpesvirus PRV.

10. Impfstoff nach einem der Ansprüche 1 bis 7, dadurch charakterisiert, dass er mindestens einen Vektor umfasst, welcher mindestens ein Gen eines Influenzavirus enthält und exprimiert.

11. Impfstoff nach Anspruch 10, dadurch charakterisiert, dass der Vektor ein Gen eines Influenzavirus exprimiert, ausgewählt aus der Gruppe bestehend aus Pferdegrippevirus, Vogelgrippevirus und Schweinegrippevirus.

12. Impfstoff nach einem der Ansprüche 1 bis 7, bestimmt zur Impfung eines Tieres der Pferdeart und umfassend einen viralen Vektor, welcher ein Gen des Pferdeherpesvirus oder ein Gen des Influenzavirus enthält.

13. Impfstoff nach Anspruch 12, dadurch charakterisiert, dass er ein Gen des Pferdeherpesvirus EHV-1 und/oder EHV-4 umfasst.

14. Impfstoff nach Anspruch 12, dadurch charakterisiert, dass er ein Gen eines Pferdeinfluenzavirus umfasst.

15. Impfstoff nach einem der Ansprüche 1 bis 14, dadurch charakterisiert, dass der virale Vektor ausgewählt ist aus der Gruppe bestehend aus Pockenvirus, Adenovirus und Herpesvirus.

16. Impfstoff nach Anspruch 15, dadurch charakterisiert, dass das Pockenvirus ausgewählt ist aus der Gruppe bestehend aus Kuh-, Geflügel-, Kanarien-, Tauben-, Schweinepockenvirus.

17. Impfstoff nach Anspruch 16, dadurch charakterisiert, dass das Kuhpockenvirus das NYVAC ist.

18. Impfstoff nach Anspruch 13, dadurch charakterisiert, dass er einen Kanarienpockenvektor umfasst, welcher die Gene gB, gC und gD des Pferdeherpesvirus EHV-1 und/oder EHV-4 enthält und exprimiert.

19. Impfstoff nach Anspruch 14, dadurch charakterisiert, dass er zwei oder drei Kanarienpockenvektoren umfasst, wobei jeder ein Gen. HA des Pferdeinfluenzavirus umfasst und jedes HA-Gen aus einem unterschiedlichen Stamm hervorgeht.

20. Impfstoff nach Anspruch 14, dadurch charakterisiert, dass er einen Kanarienpockenvektor umfasst, umfassend zwei oder drei HA-Gene aus verschiedenen Stämmen des Pferdeinfluenzavirus.

21. Impfstoff nach Anspruch 19 oder 20, dadurch charakterisiert, dass er zwei oder drei HA-Gene umfasst, welche aus den Stämmen Prag, Kentucky und/oder Newmarket stammen.

22. Impfstoff nach einem der Ansprüche 16, 18 bis 21, dadurch charakterisiert, dass der Kanarienpockenvektor ein ALVAC-Vektor ist.

23. Impfstoff nach einem der Ansprüche 1 bis 7, bestimmt zur Impfung eines Tieres der Geflügelart gegen das Influenzavirus und umfassend einen viralen Vektor, welcher ein Gen des Influenzavirus enthält.

24. Impfkit, umfassend unabhängig konditioniert einerseits in lyophilisierter Form einen rekombinanten Lebendimpfstoff, umfassend einen viralen Vektor, welcher *in vivo* eine heterologe Nucleotidsequenz enthält und exprimiert, ausgewählt aus einem Gen eines tierischen Herpesvirus und einem Gen eines Influenzavirus, vorzugsweise wie in einem der Ansprüche 8 bis 23 definiert, und andererseits, eine Lösung mindestens eines Adjuvans, ausgewählt aus den Polymeren der Acrylsäure oder Methacrylsäure und den Copolymeren von Maleinsäureanhydrid und Alkenylderivaten wie in einem der Ansprüche 1 bis 7 definiert, wobei die Adjuvanslösung dazu bestimmt ist, den lyophilisierten Impfstoff wieder aufzunehmen.

25. Verwendung einer Zusammensetzung, ausgewählt aus den Polymeren der Acrylsäure oder Methacrylsäure und den Copolymeren von Maleinsäureanhydrid und Alkenylderivaten wie in einem der Ansprüche 1 bis 7 definiert, und eines viralen Vektors, welcher *in vivo* die Nucleotidsequenz eines Gens eines tierischen Herpesvirus umfasst und exprimiert, zur Herstellung eines adjuvanten rekombinanten Impfstoffs, bestimmt zum Schutz eines Tieres gegenüber der Krankheit, welche durch dieses Herpesvirus ausgelöst ist.

26. Verwendung nach Anspruch 25, dadurch charakterisiert, dass das Gen ein Gen eines Pferdeherpesvirus EHV-1 und/oder EHV-4 ist, zur Herstellung eines adjuvanten rekombinanten Lebendimpfstoffs, bestimmt zum Schutz eines Tieres der Pferdeart gegenüber einer Krankheit, welche durch dieses Herpesvirus ausgelöst ist.

27. Verwendung einer Zusammensetzung, ausgewählt aus den Polymeren der Acrylsäure oder Methacrylsäure und den Copolymeren von Maleinsäureanhydrid und Alkenylderivaten, wie in einem der Ansprüche 1 bis 7 definiert, und eines viralen Vektors, welcher *in vivo* die Nucleotidsequenz eines Influenzavirusgens enthält und exprimiert, zur Herstellung eines rekombinanten Lebendimpfstoffs, bestimmt zum Schutz eines Tieres gegen die Grippe.

28. Verwendung nach Anspruch 27, dadurch charakterisiert, dass das Gen ein Gen des Pferdeinfluenzavirus ist, zur Herstellung eines adjuvanten rekombinanten Lebendimpfstoffs, bestimmt zum Schutz eines Tieres der Pferdeart gegen die Pferdegrippe.

29. Verwendung einer Zusammensetzung, ausgewählt aus den Polymeren der Acrylsäure oder Methacrylsäure und den Copolymeren von Maleinsäureanhydrid und Alkenylderivaten, wie in einem der Ansprüche 1 bis 7 definiert, und eines viralen Vektors, welcher *in vivo* die Nucleotidsequenz eines Gens eines Pferdeherpesvirus enthält und exprimiert, zur Herstellung eines adjuvanten rekombinanten Lebendimpfstoffs, welcher ermöglicht, bei einem Tier der Pferdeart eine signifikative Verringerung der viralen Exkretion im Vergleich zum Pferdeherpesvirus zu verursachen.

30. Verwendung einer Zusammensetzung, ausgewählt aus den Polymeren der Acrylsäure oder Methacrylsäure und den Copolymeren von Maleinsäureanhydrid und Alkenylderivaten, wie in einem der Ansprüche 1 bis 7 definiert, und eines viralen Vektors, welcher *in vivo* die Nucleotidsequenz eines Influenzavirusgens enthält und exprimiert, zur Herstellung eines adjuvanten rekombinanten Lebendimpfstoffs, welcher ermöglicht, bei einem Tier der Pferdeart eine signifikative Verringerung der viralen Exkretion im Vergleich zum Influenzavirus zu verursachen.

31. Verwendung einer Zusammensetzung, ausgewählt aus den Polymeren der Acrylsäure oder Methacrylsäure und den Copolymeren von Maleinsäureanhydrid und Alkenylderivaten, wie in einem der Ansprüche 1 bis 7 definiert, und eines viralen Vektors, welcher *in vivo* die Nucleotidsequenz eines Influenzavirusgens enthält und exprimiert, zur Herstellung eines adjuvanten rekombinanten Lebendimpfstoffs, welcher ermöglicht, bei einem Tier der Pferdeart die verfrühte Produktion von Antikörpern gegen das Influenzavirus zu verursachen.

32. Verwendung nach Anspruch 31, dadurch charakterisiert, dass es sich um eine Antikörperproduktion handelt, welche nach einer einzigen Verabreichung beobachtet wird.

33. Verwendung einer Zusammensetzung, ausgewählt aus den Polymeren der Acrylsäure oder Methacrylsäure und den Copolymeren von Maleinsäureanhydrid und Alkenylderivate, wie in einem der Ansprüche 1 bis 7 definiert, und eines viralen Vektors, welcher *in vivo* die Nucleotidsequenz eines Gens eines Influenzavirus enthält und exprimiert, zur Herstellung eines adjuvanten rekombinanten Lebendimpfstoffs, bestimmt zum Schutz eines Tieres der Vogelart gegen die Vogelgrippe.

34. Verwendung nach einem der Ansprüche 25 bis 33, dadurch charakterisiert, dass als Adjuvans ein Carbomer verwendet wird.

35. Verwendung nach einem der Ansprüche 25 bis 34, dadurch charakterisiert, dass der virale Vektor ein Pockenvirus ist, vorzugsweise ausgewählt aus Kuh-, Geflügel-, Kanarien-, Tauben- und Schweinepockenvirus.

36. Verwendung nach Anspruch 28, 30, 31 oder 32, dadurch charakterisiert, dass der virale Vektor ein Kanarienpockenvektor ist, umfassend ein HA-Gen des Influenzavirus.

37. Verwendung nach Anspruch 36, dadurch charakterisiert, dass zwei oder drei Kanarienpockenvektoren verwendet werden, umfassend ein HA-Gen des Pferdeinfluenzavirus, wobei jedes Gen aus einem unterschiedlichen Stamm hervorgeht.

38. Verwendung nach Anspruch 37, dadurch charakterisiert, dass es sich um die Stämme Prag, Kentucky und Newmarket handelt.

39. Verwendung nach Anspruch 26 oder 29, dadurch charakterisiert, dass der virale Vektor ein Kanarienpockenvektor ist, umfassend die Gene gB, gC und gD des EHV-1 und/oder EHV-4.
